Europäisches Patentamt

(19) European Patent Office

Office européen des brevets

(11) Numéro de publication: **0 132 209**
**B1**

## (12) FASCICULE DE BREVET EUROPÉEN

(45) Date de publication du fascicule du brevet:
29.04.87

(21) Numéro de dépôt: **84420107.9**

(22) Date de dépôt: **22.06.84**

(51) Int. Cl.⁴: **A 01 J 7/00,** G 01 F 5/00, G 01 N 1/20, G 01 N 23/22

(54) **Dispositif pour le prélèvement et la mesure du débit d'un liquide.**

(30) Priorité: **30.06.83 FR 8311121**

(43) Date de publication de la demande:
**23.01.85 Bulletin 85/4**

(45) Mention de la délivrance du brevet:
**29.04.87 Bulletin 87/18**

(84) Etats contractants désignés:
**AT BE CH DE FR GB IT LI LU NL SE**

(56) Documents cité:
**EP-A-0 057 816**
**DE-A-2 932 246**
**DE-B-1 247 048**
**FR-A-1 424 746**
**GB-A-1 095 708**
**GB-A-1 139 460**
**GB-A-2 072 624**
**US-A-3 045 493**

(73) Titulaire: **Savoyet, Jean- Louis Paul Jules, La Mayrie, F-38770 La Motte d'Aveillans (FR)**

(72) Inventeur: **Savoyet, Jean- Louis Paul Jules, La Mayrie, F-38770 La Motte d'Aveillans (FR)**

(74) Mandataire: **Kremer, Robert A.M., BUREAU D.A. CASALONGA OFFICE JOSSE & PETIT Morassistrasse 8, D-8000 München 5 (DE)**

LIBER, STOCKHOLM 1987

## Description

La présente invention concerne un dispositif pour le prélèvement et la mesure du débit d'un liquide en circulation constante ou pulsée et s'applique tout particulièrement bien aux liquides moussants, le lait par exemple.

On connaît déjà d'après le document EP-A-0 057 816, un dispositif répondant à cet usage. Dans un tel dispositif le lait arrive tangentiellement dans une sorte de cyclone avec convergeant à sa base pour séparer le lait de la mousse par centrifugation. Ensuite un divergent arrête la rotation du lait qui passe sous une cloche et monte dans une chambre de mesure comportant une fente verticale d'évacuation et un moyen de mesure de niveau d'équilibre, lequel s'établit automatiquement de telle manière que la section intéressée de la fente correspondant à cette hauteur permette le libre écoulement du débit moyen par unité de temps du lait qui traverse l'appareil.

Il faut remarquer que la chambre de mesure constitue une chambre d'équilibre et non une chambre d'accumulation, de sorte qu'elle ne permet pas le prélèvement ultérieur d'un échantillon de qualité moyenne. En outre tout le débit traverse cette chambre et la fente. Enfin le libre écoulement d'un liquide à travers une fente n'obéit pas à des lois extrêmement précises, et pour connaître la quantité de lait délivrée, par exemple par une vache déterminée, il faut faire intervenir une mesure précise du temps en plus de la mesure de hauteur, et intégrer celle-ci en fonction du temps, ce qui est difficile et imprécis.

C'est pourquoi on s'est orienté dans ce domaine vers d'autres dispositifs opérant selon une autre méthode qui consiste à prélever en continu un échantillon dont le débit est constamment proportionnel au débit principal dont il représente une faible fraction, et à accumuler cet échantillon dans une chambre de mesure dont le niveau monte donc progressivement au cours d'une mesure. Le volume total est alors obtenu avec une plus grande précision, indépendamment du temps, par simple proportionnalité à partir du volume de l'échantillon.

Enfin l'échantillon prélevé est représentatif de la qualité moyenne et peut être analysé par la suite.

Parmi ces derniers dispositifs, on connaît notamment, d'après le document GB-A-1 139 460, un dispositif selon le préambule de la présente revendication 1.

Cependant ce dispositif présente deux défauts principaux. D'une part sa mise en oeuvre nécessite une horizontalité parfaite pour que le facteur de proportionnalité entre le débit et le volume de l'échantillon prélevé soit constant. Il s'agit là d'une condition difficile à assurer d'une façon simple dans une étable. D'autre part, le volume de l'échantillon étant déterminé par lecture du niveau à travers un verre gradué, cette lecture est très difficile et imprécise pour un liquide moussant qui ne présente pas de surface de séparation franche. Il n'est donc à plus forte raison pas question d'intégrer cet appareil dans une chaîne de mesure avec traitement informatique.

Le but de l'invention est d'améliorer les dispositifs de ce dernier type pour en éliminer les inconvénients qui précèdent.

L'invention, définie par la partie caractérisante de la revendication 1, consiste pour l'essentiel à combiner plusieurs ouvertures de prélèvement réparties autour de l'axe, avec un moyen électrique continu effectuant une mesure relative, avec étalonnage préalable, d'une grandeur physique sensible au niveau de la partie liquide et non à celui de la mousse superficielle.

D'autres particularités de l'invention apparaîtront dans la description qui va suivre de deux modes de réalisation pris comme exemples et représentés sur le dessin annexé, sur lequel:

- la figure 1 est une vue en perspective, du haut et partiellement écorchée, des moyens de prélèvement du lait de traite, dans la première forme d'exécution, et du réceptacle;

- la figure 2 est une vue en coupe de ce même dispositif, la mesure de hauteur de l'échantillon dans le réceptacle étant du type diélectrique;

- la figure 3 est une vue en coupe et en perspective des moyens de prélèvement, dans la deuxième forme d'exécution et représentés complètement avec ses vannes de contrôle;

- la figure 4 est une vue de détail de la figure 3, au niveau du plan P-P;

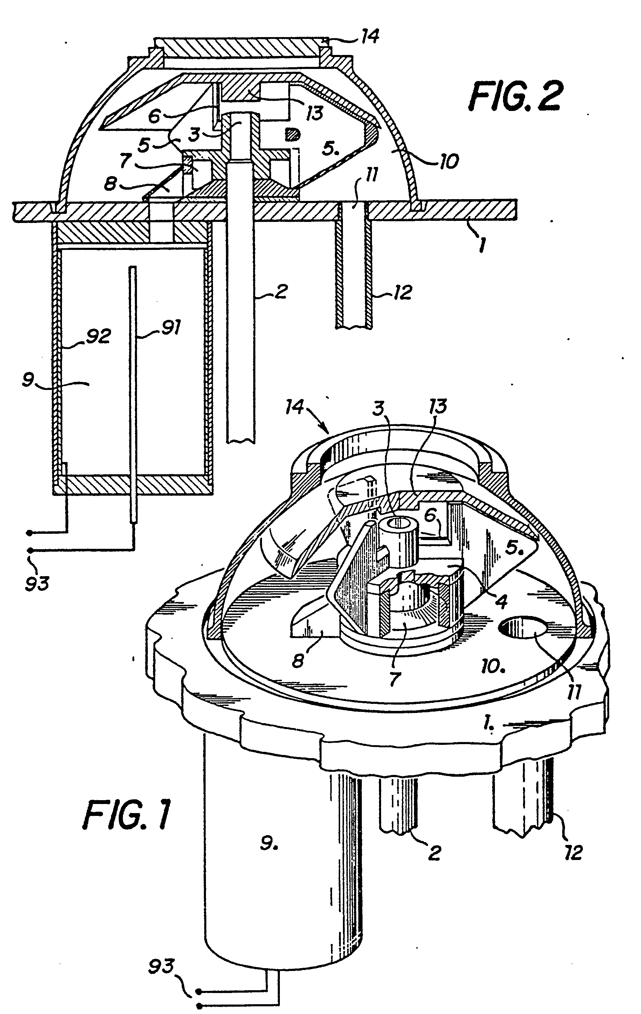

Sur la figure 1 on voit le dispositif avec son socle 1 servant de support à l'ensemble. Une tubulure d'entrée 2 amène le lait jusqu'à un orifice de sortie 3 d'où il s'écoule sur une surface en forme de dôme 4. Les moyens de prélèvement proprement dits sont constitués de couteaux 5 munis de fines fentes 6 débouchant sur une chambre 7.

Une partie du lait est prélevé par ces fentes 6 avant de s'écouler sur le dôme 4 et tombe dans la chambre 7 d'où, par l'intermédiaire d'une tubulure 8, il s'écoule dans le réceptacle 9.

Le lait non prélevé, après avoir coulé sur le dôme 4 se rassemble dans une chambre 10 et, par l'intermédiaire de l'orifice de sortie 11, passe dans la tubulure de sortie 12.

Les moyens pour répartir uniformément l'écoulement du liquide vers le dôme 4 sont constitués d'un obstacle 13 situé à légère distance de l'orifice d'entrée 3. Enfin, le tout est fermé par un couvercle 14 prenant appui sur le support 1.

Les couteaux 5 sont au nombre de trois, disposés régulièrement à 120°. On comprendra ainsi qu'une inclinaison du dispositif est sans influence sensible sur le prélèvement, puisque la quantité prélevée par les couteaux "plus bas" compense la quantité prélevée par les couteaux "plus hauts".

Comme on le voit sur cette figure 1, et encore mieux sur la figure 2, le réceptacle 9 présente une anode centrale 91 et sa paroi périphérique est recouverte d'un revêtement conducteur 92, le circuit électrique étant placé en position 93.

Cette position 93 est reliée à une logique de calcul qui déduit de la mesure diélectrique la hauteur du lait dans le réceptacle 9, donc son volume.

De préférence, à ces moyens de calcul sont associés des moyens d'affichage, de façon à obtenir par simple lecture le volume du lait prélevé.

En variante, on rencontre également des moyens de calcul et de mémoire permettant de stocker les informations et de faire des calculs statistiques, par vache traite, par paysan propriétaire ou encore par biotope.

Dans un autre mode d'exécution, les électrodes 91 et 92 du réceptacle 9 sont remplacées par un capteur de pression noyé dans le fond et mesurant la presion exercée sur lui par la colonne de liquide. Les légères déformations engendrent un signal électrique variable selon l'amplitude de ces déformations, signal qui est traité par une logique pour livrer le volume de l'échantillon prélevé.

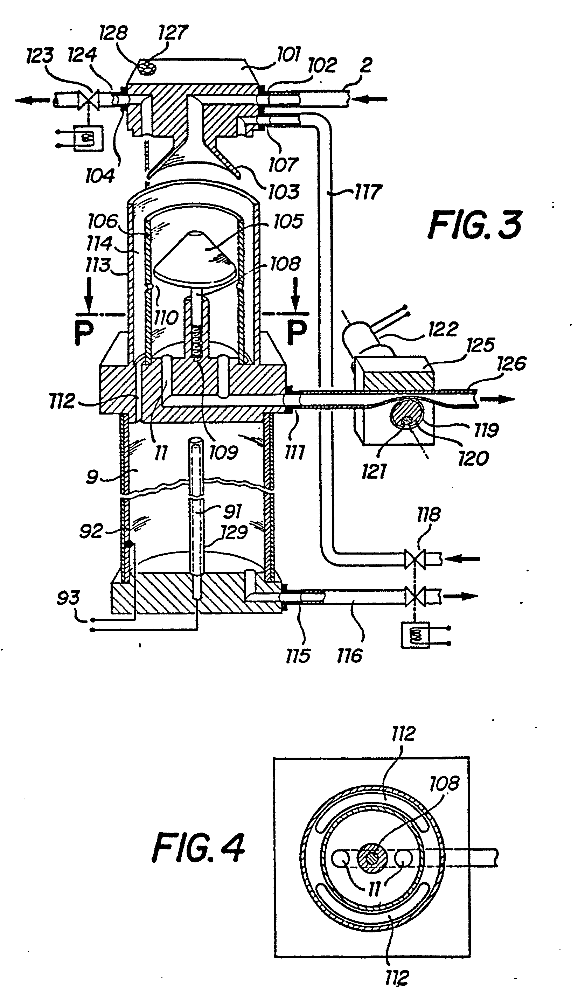

Sur la figure 3, qui correspond à la deuxième forme d'exécution, on voit une tête 101 pour l'admission du lait par l'intermédiaire de la tubulure 102, qui se termine en forme d'entonnoir renversé 103. On notera également une tubulure 104 pour le nettoyage, ainsi qu'une tubulure 107 de prise d'air.

Cette tête 101 forme couvercle et vient se loger, au niveau de l'entonnoir 103, dans une chambre cylindrique 106 juste au-dessus d'une surface conique 105, de façon à ce que le lait admis soit distribué uniformément. Cette surface conique 105 se prolonge, selon son axe, par une tige de maintien 108, qui repose dans un logement sur un ressort 109.

La pression du lait admis contrebalance l'effet du ressort 109 de rappel, de sorte qu'un espace suffisant est maintenu entre l'entonnoir 103 et la surface conique 105. En variante, le ressort peut être supprimé, l'axe supportant le cône continuant ou non à être mobile.

Dans les parois de la chambre cylindrique 106 sont ménagées des ouvertures latérales 110, dont l'axe est sensiblement parallèle à celui de la surface conique 105 et qui sont disposées juste immédiatement au-dessous de celle-ci.

Le lait non prélevé, c'est-à-dire ne passant pas par les ouvertures latérales 110, est récupéré par les orifices d'évacuation 11, qui se réunissent en une tubulure de sortie 111.

Le lait prélevé quant à lui, passe dans les trous oblongs 112 illustrés en détails sur la figure 4, pour être collecté dans le réceptacle 9. En variante non représentée, les trous oblongs peuvent être remplacés par une ou plusieurs tubulures.

On remarquera que la chambre cylindrique 106 est entourée d'une autre chambre cylindrique 113, ce qui définit un espace annulaire 114 par où passe un liquide de nettoyage ou de rinçage du réceptacle 9, liquide qui est admis par la tubulure 104. Une tubulure 115 sert à la vidange du réceptacle 9.

Les tubulures 104, 111 et 115 sont munies de tuyaux flexibles, respectivement 124, 126 et 116, permettant ainsi un pincement. Le dispositif au complet comporte avantageusement trois vannes, la vanne 125 contrôlant la sortie du lait, la double vanne 118 contrôlant la vidange du réceptacle 9 et la vanne 123 permettant le nettoyage.

La fermeture des vannes s'effectue par l'intermédiaire d'un motoréducteur 122, un axe 121 effectuant un quart de tour ou un demi-tour, ce qui entraîne en rotation un excentrique 120; le pincement est alors effectué, comme on le voit sur la figure 3.

On peut constater que l'excentrique 120 est muni d'une bague 119, qui a pour but de réaliser un pincement élastique et permet d'éviter l'arrachement et la détérioration du tuyau flexible 126.

Lors de la traite, la vanne 125 est ouverte et les vannes 118 et 123 sont fermées. Le lait arrivant par la tubulure 102 s'écoule sur le cône 105, une partie de celui-ci est prélevée par l'intermédiaire des ouvertures latérales 110, comme explicité plus haut et s'écoule dans le réceptacle 9 par l'intermédiaire des ouvertures 112. L'autre partie s'écoule par les ouvertures 11 dans la tubulure 111.

Après la traite, la vanne 125 peut être fermée et la double vanne 118 est ouverte, permettant ainsi la prise d'air par la tubulure 107. L'écoulement du lait peut maintenant s'effectuer par la tubulure 115.

Pour le nettoyage, on fait entrer un liquide de nettoyage par la tubulure 102. Les vannes 125 et 118 étant fermées et la vanne 123 ouverte, le liquide de nettoyage remplit alors l'appareil et le tout s'écoule par la vanne 123 lorsque l'ensemble est plein. Après un laps de temps géré par l'électronique, les vannes 118 et 125 s'ouvrent, permettant ainsi la vidange de l'appareil. Cette opération peut être effectuée à plusieurs reprises, mais doit être suivie de préférence d'une opération de rinçage selon la méthode décrite ci-après.

Le liquide de rinçage entre dans l'appareil par la tubulure 102, le remplit, et s'ecoule par la tubulure 123. Après un laps de temps, les vinnes 118 et 125 sont ouverts, permettant ainsi la vidange de l'appareil.

En variante, le liquide de nettoyage entre dans l'appareil par la tubulure 102, les vannes 125 et 118 étant fermées et la vanne 123 est ouverte; le liquide de nettoyage remplit le tout puis s'écoule par la vanne 123. Après un laps de temps géré par l'électronique, la vanne 125 est ouverte, permettant ainsi la vidange de la partie mesure. La vanne 125 est de nouveau fermée, l'appareil de nouveau rempli, et après un laps de temps, les vannes 125 et 118 sont ouvertes, permettant ainsi la vidange totale de l'appareil.

On répète alors cette opération avec le liquide de rinçage.

## Revendications

1. Dispositif pour le prélèvement d'un échantillon et la mesure du débit d'un liquide en circulation constante ou pulsée, notamment d'un liquide moussant tel que du lait, comprenant un tube d'entrée du liquide (2; 102) d'axe sensiblement vertical, un distributeur (3, 13; 103, 105) répartissant l'écoulement de ce liquide en un film liquide relativement étendu et uniformément réparti autour dudit axe, ledit film s'écoulant sur un dôme (4; 105), avec une ouverture de prélèvement (6; 110) disposée dans le trajet de ce film pour en intercepter une fraction connue, cette ouverture conduisant l'échantillon ainsi prélevé par une tube de sortie d'échantillon (8; 112) à un réceptacle (9) permettant sa mesure de volume et éventuellement sa reprise, la partie non prélevée du débit s'écoulant par un orifice d'évacuation (11) et le débit total étant évalué par proportionnalité à partir du volume de l'échantillon, caractérisé par le fait
- qu'il comporte plusieurs desdites ouvertures de prélèvement (6; 110) régulièrement réparties angulairement autour dudit axe pour compenser les erreurs de verticalité de cet axe, et
- que ladite mesure de volume de l'échantillon dans le réceptacle (9) est assurée par un moyen électrique continu effectuant une mesure relative, avec étalonnage préalable, d'une grandeur physique sensible au niveau de la partie liquide et non à celui de la mousse superficielle éventuelle.

2. Dispositif selon la revendication 1, caractérisé par le fait que lesdites ouvertures de prélèvement (6) sont constituées par des fentes situées chacune dans le bord intérieur d'une cloison radiale (5) en forme de couteau, ces cloisons étant également réparties angulairement autour dudit axe.

3. Dispositif selon la revendication 2, caractérisé par le fait que les cloisons (5) et les ouvertures (6) sont au nombre de trois disposées à 120° l'une de l'autre.

4. Dispositif selon la revendication 1, caractérisé par le fait que le distributeur est constitué par un intervalle étroit ménagé entre une tête (103) en forme d'entonnoir renversé et un dôme (105) de forme conique, et que les ouvertures de prélèvement sont constituées par des ouvertures latérales (110) percées dans la paroi d'une chambre cylindrique (106) entourant le distributeur.

5. Dispositif selon la revendication 4, caractérisé en ce qu'il comprend au moins deux ouvertures latérales (110) d'axe sensiblement parallèle à la surface conique (105) et disposées immédiatement au-dessous de celle-ci.

6. Dispositif selon la revendication 4, caractérisé en ce que la surface conique (105) est fixée axialement sur des moyens élastiques (109) de rappel agissant à l'encontre de son enfoncement dans la chambre cylindrique (106).

7. Dispositif selon la revendication 4, caractérisé en ce que les moyens élastiques (106) sont constitués d'un ressort.

8. Dispositif selon la revendication 4, caractérisé en ce que la chambre cylindrique (106) est entourée d'une autre chambre cylindrique (113), l'espace annulaire (114) ménagé entre les deux chambres servant à admettre un liquide de nettoyage du réceptacle (9).

9. Dispositif selon la revendication 1, caractérisé en ce que le moyen électrique mesure le poids de la colonne de liquide présent dans le réceptacle (9).

10. Dispositif selon la revendication 9, caractérisé en ce que le moyen électrique comprend un capteur de pression dont les légères déformations engendre un signal électrique variable selon l'amplitude de ces déformations.

11. Dispositif selon la revendication 1, caractérisé en ce que le moyen électrique mesure la constante diélectrique du liquide présent dans le réceptacle (9).

12. Dispositif selon la revendication 11, caractérisé en ce que le moyen électrique comprend une anode centrale (91) s'étendant verticalement depuis le fond du réceptacle (9), la seconde électrode étant constituée par la paroi périphérique du réceptacle au par un revêtement (92) apposé sur celle-ci.

13. Dispositif selon la revendication 1, caractérisé en ce qu'il comprend des moyens d'affichage et une logique de calcul du volume en fonction du signal électrique reçu, en vue de l'affichage direct dudit volume.

14. Dispositif selon la revendication 1, caractérisé en ce qu'il comprend outre les circuits d'entrée et de sortie du liquide, un circuit pour la vidange du réceptacle (9) et un circuit de rinçage et de nettoyage.

15. Dispositif selon la revendication 1, caractérisé en ce que tous les circuits sont constitués par des tubulures souples et contralés par des vannes agissant directement sur ces tubulures.

16. Utilisation du dispositif selon la revendication 1 pour le lait de traite.

## Patentansprüche

1. Vorrichtung zur Probennahme und Durchflußmessung einer konstant zirkulierenden oder gepulsten Flüssigkeit, insbesondere einer schäumenden Flüssigkeit, wie Milch, mit einem Flüssigkeitseinlaßrohr (2; 102) mit im wesentlichen senkrechter Achse, einem Verteiler (3, 13; 103, 105), welcher den Flüssigkeitsstrom zu einem verhältnismäßig ausgedehnten und gleichmäßig um die Achse verteilten Flüssigkeitsfilm verteilt, welcher sich über eine Haube (4; 105) mit einer im Weg dieses Films angeordneten Entnahmeöffnung (6; 110) zum Auffangen einer bekannten Fraktion ergießt, wobei diese Öffnung die so entnommene Probe durch ein Probenauslaßrohr (8; 112) zu einem ihre Volumsmessung und gegebenenfalls ihre Wiederaufnahme erlaubenden Behälter (9) leitet, und sich der nicht entnommene Teil des Durchsatzes durch eine Entleerungsöffnung (11) ergießt und der Gesamtdurchsatz proportional vom Probenvolumen ausgehend ausgewertet wird, dadurch gekennzeichnet,

- daß sie mehrere Entnahmeöffnungen (6; 110) gleichmäßig im Winkel um die Achse verteilt umfaßt, um die Vertikalitätsfehler dieser Achse auszugleichen, und

- daß die Volumsmessung der Probe im Behälter (9) durch ein eine relative Messung mit vorhergehender Eichung einer in der Höhe des flüssigen Teils und nicht in der etwaigen oberflächlichen Schaums wahrnehmbaren physikalischen Größe durchführendes Gleichstromgerät sichergestellt ist.

2. Vorrichtung nach Anspruch 1, dadurch gekennzeichnet, daß die Entnahmeöffnungen (6) aus jeweils am Innenrand einer radialen laschenförmigen Zwischenwand (5) angeordneten Schlitzen bestehen, wobei diese Zwischenwände ebenfalls im Winkel um die Achse verteilt sind.

3. Vorrichtung nach Anspruch 2, dadurch gekennzeichnet, daß die Zwischenwände (5) und die Öffnungen (6) jeweils drei und um 120° voneinander angeordnet sind.

4. Vorrichtung nach Anspruch 1, dadurch gekennzeichnet, daß der Verteiler aus einem zwischen einem Kopf (103) in Form eines umgekehrten Trichters und einer Haube (105) konischer Form angebrachten engen Abstand besteht und daß die Entnahmeöffnungen aus in die Wand einer den Verteiler umgebenden zylindrischen Kammer (106) gebohrten seitlichen Öffnungen (110) bestehen.

5. Vorrichtung nach Anspruch 4, dadurch gekennzeichnet, daß sie mindestens zwei seitliche Öffnungen (110), welche eine zur konischen Oberfläche (105) im wesentlichen parallele Achse haben und unmittelbar unterhalb jener angeordnet sind, aufweist.

6. Vorrichtung nach Anspruch 4, dadurch gekennzeichnet, daß die konische Oberfläche (105) axial an entgegen ihrer Versenkung in die zylindrische Kammer (106) wirkenden elastischen Rückholmitteln (109) befestigt ist.

7. Vorrichtung nach Anspruch 4, dadurch gekennzeichnet, daß die elastischen Rückholmittel (106) aus einer Feder bestehen.

8. Vorrichtung nach Anspruch 4, dadurch gekennzeichnet, daß die zylindrische Kammer (106) von einer anderen zylindrischen Kammer (113) umgeben ist, wobei der zwischen den beiden Kammern freigelassene ringförmige Raum (114) zum Einbringen einer Flüssigkeit zur Reinigung des Behälters (9) dient.

9. Vorrichtung nach Anspruch 1, dadurch gekennzeichnet, daß das Gleichstromgerät das Gewicht der im Behälter (9) vorhandenen Flüssigkeitssäule mißt.

10. Vorrichtung nach Anspruch 9, dadurch gekennzeichnet, daß das Gleichstromgerät eine Druckmeßzelle, deren leichte Verformungen ein gemäß der Amplitude dieser Verformungen variables elektrisches Signal erzeugen, aufweist.

11. Vorrichtung nach Anspruch 1, dadurch gekennzeichnet, daß das elektrische Gerät die Dielektrizitätskonstante der im Behälter (9) vorhandenen Flüssigkeit mißt.

12. Vorrichtung nach Anspruch 11, dadurch gekennzeichnet, daß das elektrische Gerät eine zentrale Anode (91) umfaßt, welche sich vertikal vom Boden des Behälters (9) erstreckt, wobei die zweite Elektrode aus einer Umfangswand des Behälters oder aus einer auf diese aufgebrachten Beschichtung (92) besteht.

13. Vorrichtung nach Anspruch 1, dadurch gekennzeichnet, daß sie eine Sichtanzeige und eine Logik zur Berechnung des Volumens in Abhängigkeit des empfangenen elektrischen Signals im Hinblick auf die Direktanzeige des Volumens umfaßt.

14. Vorrichtung nach Anspruch 1, dadurch gekennzeichnet, daß sie außer den Einlaß- und Auslaßkreisen der Flüssigkeit einen Kreis für die Entleerung des Behälters (9) und einen Kreis für die Spülung und die Reinigung umfaßt.

15. Vorrichtung nach Anspruch 1, dadurch gekennzeichnet, daß alle Kreise aus biegsamen und von direkt auf sie einwirkenden Ventilen gesteuerten Rohrsystemen bestehen.

16. Anwendung einer Vorrichtung nach Anspruch 1 für Melkmilch.

## Claims

1. Apparatus for extracting a sample and measuring the flow of a liquid in constant or pulsed circulation, especially of a foaming liquid, such as milk, comprising a liquid inlet pipe (2; 102) of substantially vertical axis, a distributor (3, 13; 103, 105) distributing the flow of this liquid in a relatively extensive liquid film uniformly distributed about the said axis, the said film flowing over a dome (4; 105) with an extraction aperture (6; 110) arranged in the path of this film in order to intercept a known fraction of it, this aperture leading the sample thus extracted via a sample outlet pipe (8; 112) to a vessel (9) making it possible to measure its volume and, if appropriate, collect it, the portion of the flow not extracted flowing off via a discharge orifice (11), and the total flow being evaluated by proportionality from the volume of the sample, characterized in that it comprises several of the said extraction apertures (6; 110) uniformly distributed angularly about the said axis, in order to compensate the verticality errors of this axis, and in that the said measurement of the volume of the sample in the vessel (9) is ensured by a continuous electrical means which, with prior calibration, carries out a relative measurement of a physical parameter sensitive to the level of the liquid portion and not to that of the possible surface foam.

2. Apparatus according to Claim 1, characterized in that the said extraction orifices (6) are formed by slits each located in the inner edge of a knife-shaped radial partition (5), these partitions likewise being distributed angularly about the said axis.

3. Apparatus according to Claim 2 characterized in that there are three partitions (5) and apertures (6) arranged at 120° relative to one another.

4. Apparatus according to Claim 1, characterized in that the distributor is formel by a narrow gap provided between a head (103) in the form of an upturned funnel and a dome (105) of conical shape, and in that the extraction apertures are formed by lateral apertures (110) made in the wall of a cylindrical chamber (106) surrounding the distributor.

5. Apparatus according to Claim 4, characterized in that it has at least two lateral apertures (110) with an axis substantially parallel to the conical surface, (105) and arranged immediately below the latter.

6. Apparatus according to Claim 4, characterized in that the conical surface (105) is fastened axially to elastic restoring means (109) acting counter to its penetration into the cylindrical chamber (106).

7. Apparatus according to Claim 4, characterized in that the elastic means (106) consist of a spring.

8. Apparatus according to Claim 4, characterized in that the cylindrical chamber (106) is surrounded by another cylindrical chamber (113), and the annular space (114) formed between the two chambers serves for admitting a liquid for cleaning the vessel (9).

9. Apparatus according to Claim 1, characterized in that the electrical means measure the weight of the liquid column present in the vessel (9).

10. Apparatus according to Claim 9, characterized in that the electrical means comprise a pressure sensor, the slight deformations of which generate an electrical signal variable according to the amount of the deformations.

11. Apparatus according to Claim 1, characterized in that the electrical means measures the dielectric constant of the liquid present in the vessel (9).

12. Apparatus according to Claim 11, characterized in that the electrical means comprises a central anode (91) extending vertically from the bottom of the vessel (9), and a second electrode consisting of the peripheral wall of the vessel or of a covering (92) affixed to the latter.

13. Apparatus according to Claim 1, characterized in that it comprises display means and a logic unit for calculating the volume as a function of the electrical signal received, for the purpose of the direct display of the said volume.

14. Apparatus according to Claim 1, characterized in that it comprises, in addition to the liquid inlet and outlet circuits, a circuit for emptying the vessel (9) and a rinsing and cleaning circuit.

15. Apparatus according to Claim 1., characterized in that all the circuits are formed by flexible pipes controlled by valves acting directly on these pipes.

16. Use of the apparatus according to Claim 1 for freshly-milked milk.

FIG.2

FIG.1

FIG. 3

FIG. 4